# EUROPÄISCHE PATENTSCHRIFT

(11) **EP 1 725 293 B1**
(45) Veröffentlichungstag und Bekanntmachung des Hinweises auf die Patenterteilung: **30.07.2008**
(21) Anmeldenummer: 05732028.5
(22) Anmeldetag: 11.03.2005
(51) Int. Cl.: A61M 25/10

(54) **BALLONKATHETER**
BALLOON CATHETER
CATHETER A BALLONNET

(30) Priorität: 16.03.2004 DE 102004013012
(43) Veröffentlichungstag der Anmeldung: 29.11.2006
(73) Patentinhaber: Universitätsklinikum Schleswig-Holstein, 24105 Kiel (DE)
(72) Erfinder: LUTTER, Georg, 24105 Kiel (DE)
(74) Vertreter: Biehl, Christian
(86) Internationale Anmeldenummer: PCT/DE2005/000437
(87) Internationale Veröffentlichungsnummer: WO 2005/089854

(56) Entgegenhaltungen:
- US-A- 5 423 745
- US-A1- 2002 120 234

## Beschreibung

Die Erfindung betrifft einen Ballonkatheter zum Abdichten von Blutgefäßabschnitten zur Behandlung von Gefäßkrankheiten. Insbesondere betrifft die Erfindung einen Ballonkatheter zur Verwendung bei minimalinvasiven chirurgischen Eingriffen am menschlichen Herzen.

Zu den am häufigsten durchgeführten Eingriffen der Herzchirurgie gehört die Korrektur von Herzklappenfehlern. Neben offenen Operationsmethoden, bei denen der operative Eingriff am stillstehenden, offenen und blutleeren Herzen erfolgt, bedient man sich am arbeitenden Herzen minimalinvasiver Verfahren (geschlossene Operationsmethoden). Dazu werden geeignete Werkzeuge benötigt, die derart ausgebildet, dass sie in das Blutgefäßsystem eingebracht und bis zur Operationsstelle vorgebracht werden können.

Bei der Operation am Herzen kommen spezielle Perfusionskatheter zum Einsatz, die vom Prinzip der Ballonkatheter, wie sie beispielsweise aus der DE 195 33 601 bekannt sind, Gebrauch machen. Die US 6,135,981 schlägt beispielsweise einen Perfusionskatheter mit zwei distal benachbart angeordneten, aufblasbaren Kammern vor, die einen von den Kammern eingeschlossenen Operationsraum bilden, der vom Blutkreislauf ausgenommen ist. Zur sicheren Positionierung der Ballons im Gefäß kann die Oberfläche der Ballons zusätzlich nach Art des in der US 5,423,745 vorgeschlagenen Ballonkatheters mit Oberflächenstrukturen in Form von Noppen oder spiralig oder ringförmig angeordneten Ausbuchtungen ausgebildet sein. Dies US 5 423 745 beschreibt einen Ballonkatheter gemäß Oberbegriff von Patentanspruch 1.

Für die Ablation von insuffizienten oder stenosierten Herzklappen ist insbesondere aus der DE 102 17 559 eine Vorrichtung mit zwei entlang des Katheters angeordneten, inflatierbaren Dilatationseinheiten bekannt. Die Dilatationseinheiten sind so angeordnet, dass die distal angeordnete Dilatationseinheit unterhalb, die proximal angeordnete Dilatationseinheit oberhalb der Aortenklappe zu liegen kommen. Sie bilden nach außen jeweils einen fluiddichten Abschluss mit der Gefäßwand und schaffen innenliegend einen Abschnitt mit einem blutleeren Arbeitsvolumen, innerhalb dessen der Operateur unter direkter Beobachtung die Aortenklappe unter Einsatz geeigneter Katheter-Werkzeuge behandeln kann.

Während die proximale Dilatationseinheit toroidal ausgebildet ist und zu einem optimalen Abschluss der Aorta ascendens führt, kann die Positionierung der distalen, ebenfalls toroidal ausgebildeten Dilatationseinheit aufgrund der anatomischen Verhältnisse in den Herzkammern zu Dichtungsproblemen führen.

Aufgabe der Erfindung ist es deshalb, einen Katheter bereit zu stellen, der eine zuverlässige und kontrollierbare Abdichtung von Gefäßabschnitten ermöglicht.

Die Aufgabe wird gelöst durch einen Ballonkatheter zum Abdichten von Blutgefäßen und Herzkammern mit wenigstens einer mit einer ersten Zuleitung verbundenen aufblasbaren Kammer, **gekennzeichnet durch** eine an die wenigstens eine aufblasbare Kammer angrenzende Einheit, die mit der Kammer einen Hohlraum, in den eine Unterdruckleitung mündet, bildet, wobei zwischen den den Hohlraum bildenden Wandungen Abstandselemente vorgesehen sind, und zum Ansaugen des Ballonkatheters an das Blutgefäß oder die Herzkammer auf ihrer Außenseite wenigstens in einem begrenzten Bereich gasdurchlässig ist.

Der erfindungsgemäße Katheter besitzt den Vorteil, dass durch Anlegen eines Unterdrucks an den von der Einheit und der Kammerwandung gebildeten Hohlraum ein Ansaugen des umliegenden Gewebes und damit ein formschlüssiges Abdichten des Ballonkatheters mit seiner Umgebung *in situ* erreicht wird.

Die Erfindung wird anhand von Zeichnungen näher erläutert. Es zeigen:
- Fig. 1: eine Seitenansicht des erfindungsgemäßen Ballonkatheters;
- Fig. 2: eine Draufsicht des Ballonkatheters aus Fig. 1;
- Fig. 3: einen Querschnitt in Seitenansicht durch ein bevorzugtes Ausführungsbeispiel der Erfindung;
- Fig. 4: eine Seitenansicht eines bevorzugten Ausführungsbeispiels;
- Fig. 5: einen Querschnitt in Seitenansicht des Ballonkatheters aus Fig. 4;
- Fig. 6: einen Querschnitt in Draufsicht des Ballonkatheters aus Fig. 4;
- Fig. 7: eine Seitenansicht eines bevorzugten Ausführungsbeispiels der Erfindung;
- Fig. 7a: eine Vergrößerung der Abstandselemente aus Fig. 7
- Fig. 7b: eine Vergrößerung der Abstandselemente als Alternative zu den in Fig. 7a gezeigten Abstandselementen;
- Fig. 8: einen Querschnitt durch ein menschliches Herz mit einem vom Erfindungsgegenstand Gebrauch machenden Katheter zur Aortenklappen-Ablation *in situ*
- Fig. 9: ein weiteres Anwendungsbeispiel des erfindungsgemäßen Ballonkatheters in Kombination mit einem weiteren Katheter im Querschnitt durch ein menschliches Herz *in situ*

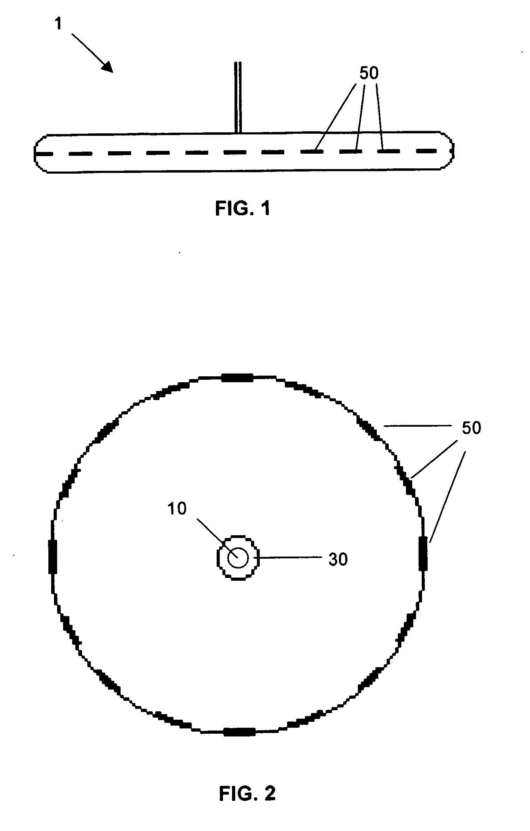

Fig. 1 zeigt eine Seitenansicht des erfindungsgemäßen Ballonkatheters 1. In der äußeren Ansicht ähnelt der erfindungsgemäße Ballonkatheter einem herkömmlichen Katheter mit einem Leitungsanteil und einem Ballonanteil mit der Ausnahme, dass ein um den Ballon umlaufender, bevorzugt unterbrochener, begrenzter Bereich 50 angeordnet ist, der gasdurchlässig ist und im Allgemeinen durch makroskopische Poren gebildet wird.

Zur Verdeutlichung zeigt Fig. 2 den Ballonkatheter aus Fig. 1 in Draufsicht. Dabei ist zu erkennen, dass in diesem bevorzugten Ausführungsbeispiel der Erfindung der Leitungsanteil des Katheters aus einer inneren, ersten Zuleitung 10 und einer die erste Zuleitung 10 umgebene Unterdruckleitung 30 besteht. Deutlich tritt auch hervor, dass die gasdurchlässigen Bereiche 50 um den Katheter umlaufend angeordnet sind.

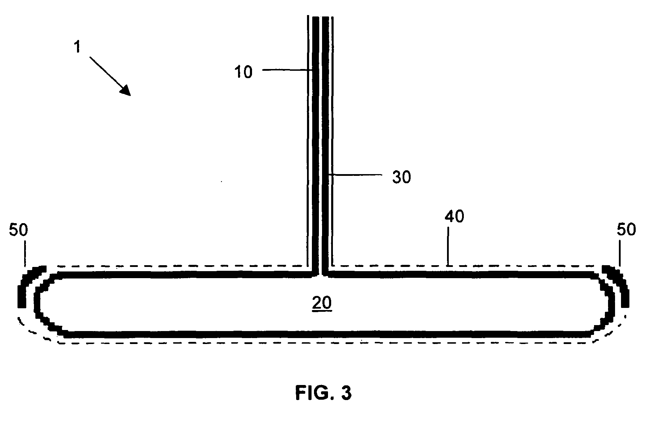

Fig. 3 zeigt ein besonders bevorzugtes Ausführungsbeispiel der Erfindung, für das auch die Fig. 1 und 2 Anwendung finden. Der Ballonkatheter besteht aus einer mit einer Zuleitung 10 verbundenen aufblasbaren Kammer 20. An die aufblasbare Kammer 20 grenzt die Einheit 40, die auf ihrer Außenseite wenigstens in einem begrenzten Bereich 50 gasdurchlässig ist, an und bildet mit der Kammer 20 einen Hohlraum, in den eine Unterdruckleitung 30 mündet. In einer bevorzugten Ausgestaltung der Erfindung umläuft der begrenzte Bereich 50 den Ballonkatheter 1 auf der Außenseite der Einheit kreisförmig und ist in regelmäßigen Abständen von gasdurchlässigen Poren durchbrochen. Fig. 1 zeigt eine bevorzugte Ausgestaltung, bei der die Unterdruckleitung 30 die erste Zuleitung 10 und die Einheit 40 die Kammer 20 umschließt.
Zur Verwendung des Ballonkatheters 1 wird die Kammer 20 in deflatiertem Zustand *in situ* positioniert und durch Einleiten eines Gases oder einer Flüssigkeit durch die Zuleitung 10 in die Kammer 20 auf ihre vollständige Größe gebracht. Mit der Ausdehnung der Kammer 20 wird auch die an die Kammer 20 angrenzende Einheit 40 gedehnt und erreicht ihre größte Ausdehnung bei maximaler Ausdehnung der Kammer 20.

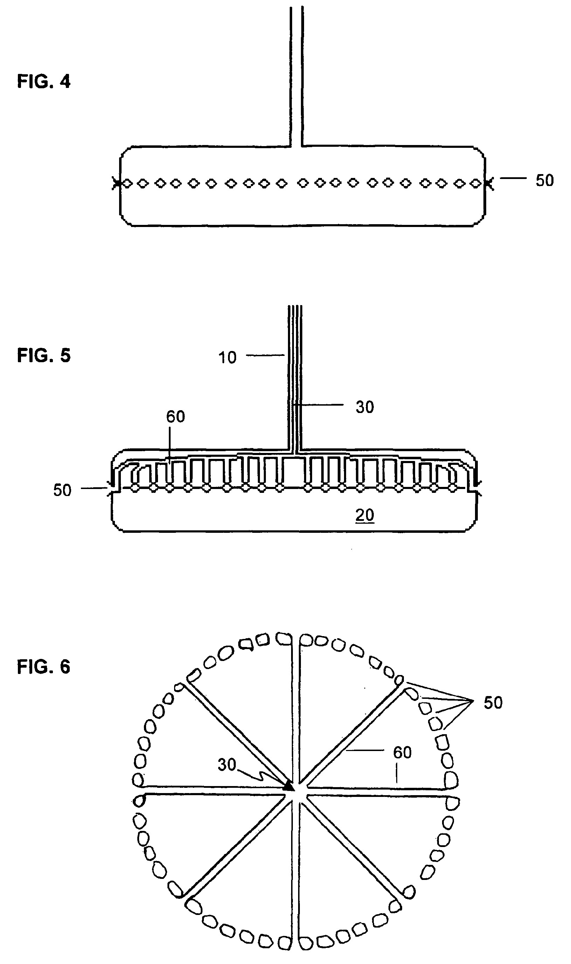

Fig. 4 zeigt ein weiteres Ausführungsbeispiel, bei dem die gasdurchlässigen Bereiche 50 nach Erreichen der maximalen Ausdehnung der Kammer 20 auf der Außenseite der Einheit 40 in trompetenartigen Ausstülpungen enden, um so ein Ansaugen des Ballonkatheters an das Gewebe zu erleichtern.

Da bei Anlegen eines Unterdrucks die Einheit 40 ohne geeignete Mittel kollabieren würde, sind zwischen den den Hohlraum bildenden Wandungen Abstandselemente vorgesehen, die ein Kollabieren verhindern. Bevorzugt bilden diese Abstandselemente die in Fig. 5 dargestellen röhrenförmige Leitungen 60, die zu den gasdurchlässig ausgebildeten Bereichen 50 führen und bevorzugt in einer Pore, wie z.B. in Fig. 4 dargestellt, nach außen münden. Das Zuleitungssystem der die Leitungen 60 bildenden Abstandselemente kann sich sinnvollerweise wie in Fig. 6 abgebildet verzweigen, um ein gleichmäßiges Ansaugen an allen gasdurchlässigen Bereichen 50 zu ermöglichen.

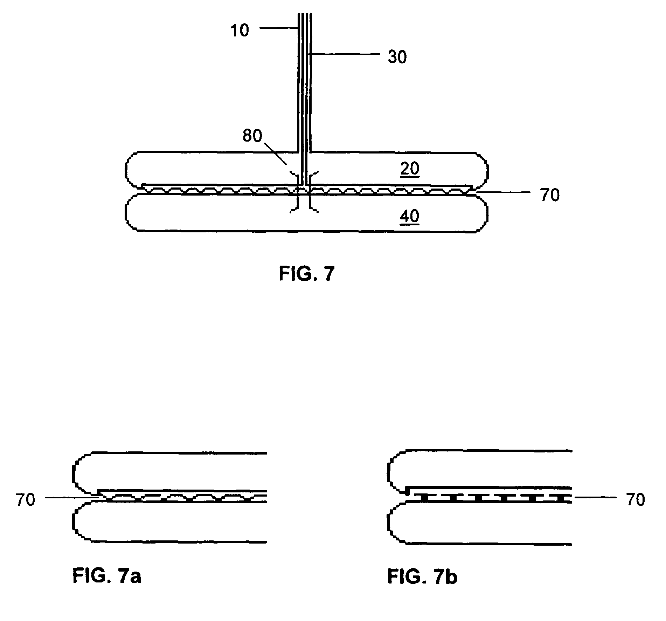

Fig. 7 zeigt eine besonders bevorzugte Ausgestaltung der Erfindung, bei der die Abstandselemente als so genannte, in Fig. 7a und Fig. 7b exemplarisch abgebildete Noppen 70 arbeiten, die entweder geschlossen oder mit Luft/Flüssigkeit gefüllt werden können, um so den Abstand zwischen der Kammer 20 und der Einheit 40 aufrecht zu erhalten, die über das Verbindungselement 80 miteinander verbunden sind.

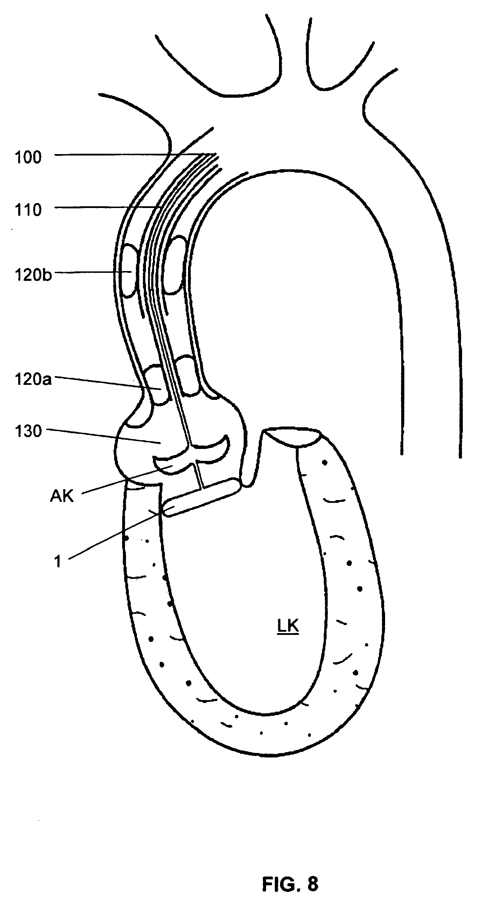

Fig. 8 zeigt ein als Katheter zur Aortenklappen-Ablation ausgebildetes Beispiel des erfindungsgemäßen Ballonkatheters. Der aus der DE 102 17 559 bekannte Katheter wurde hier mit der vorliegenden Erfindung kombiniert. Der Katheter selbst besteht aus einem Perfusionskatheter 100, einem Schleusenkanal 110 durch das die Instrumente in den Arbeitsbereich 130, der die Aortenklappe AK umschließt, eingebracht werden und mehreren Dilatationselementen 120a, 120b, 1. Das Dilatationselement 120b dient zur Unterstützung der Führung des Katheters, während das Dilatationselement 120a den Arbeitsbereich 130 nach proximal abschließt. Das distale Dilatationselement, das durch den erfindungsgemäßen Ballonkatheter 1 dargestellt wird, schließt den Arbeitsbereich 130 von der linken Herzkammer LK nach distal formschlüssig ab. Dieser Ballonkatheter 1 kann jedoch auch weiter zurück im Blutstrom, tiefer in der linken Kammer LK oder im linken Vorhof abschließen, so dass eine Blutstromunterbrechung aufrechterhalten werden kann. Dazu wird der mit dem Katheter verbundene Ballonkatheter über die Zuleitung 30 durch Anlegen eines Unterdrucks an den Hohlraum 40 an den linken Ausflusstrakt der linken Herzkammer und an der Mitralklappe fest gesogen.

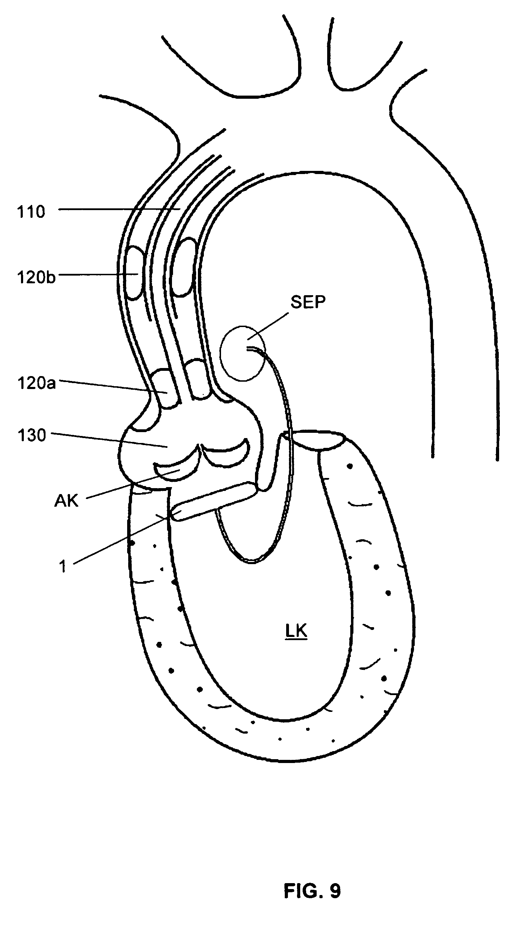

Fig. 9 zeigt ein weiteres Anwendungsbeispiel des erfindungsgemäßen Ballonkatheters in Kombination mit einem weiteren Katheter im Querschnitt durch ein menschliches Herz *in situ.* In diesem Fall besteht der aus der DE 102 17 559 bekannte Katheter aus den in Fig. 2 benannten Elementen, allerdings ohne ein distales Dilatationselement. Letzteres wird durch den nicht mit dem Katheter verbundenen erfindungsgemäßen Ballonkatheter 1 gestellt, indem es minimalinvasiv über das Septum SEP in die linke Herzkammer LK eingebracht wird. Dieses Vorgehen hat den Vorteil, dass in der reduzierten Version des Katheters zur Aortenklappen-Ablation der Schleusenkanal 110 ein wesentlich größeres Volumen zur Passage von zur Ablation benötigten Werkzeugen in den Arbeitsbereich 130 besitzt.

Zusammengefasst zeichnet sich ein Verfahren zum Herzklappenersatz mit dem erfindungsgemäßen Ballonkatheter bevorzugt durch folgende Verfahrensschritte aus:
- Anschluss einer Herz-Lungenmaschine in an sich bekannter Weise, z.B. in der Leistenregion,
- Applikation der Cardioplegie beispielsweise über die *Aorta ascendens* oder den *Sinus coronarius,*
- Einbringen und Positionieren des erfindungsgemäßen distalen Ballonkatheters am Ort des linken Ausflusstrakts der linken Herzkammer, in der linken Herzkammer oder linkem Vorhof, entweder über die Aorta durch die Herzklappe hindurch oder vorzugsweise über die Vorhofscheidewand des Herzens direkt in den Bereich der linken Herzkammer und Fixieren des Ballonkatheters durch Ansaugen an den linken Ausflusstrakt der linken Herzkammerund an der Mitralklappe,
- Einbringen und Positionieren von weiteren Kathetern zum Verschliessen der Coronararterien,
- Einbringen und Positionieren eines weiteren erfindungsgemäßen proximalen Ballonkatheters oberhalb der Aortenklappe, sodass eine Aortenablationskammer entsteht, in der in Ruhe die Aortenablation mit kathetergeführten Werkzeugen (Water Jet, Laser, Endoskop, Saugung, Haltekatheter etc.) durchgeführt werden kann.

Der Vorteil dieses Vorgehens besteht darin, dass der proximal einzuführende Katheter einen größeren Katheterhohlraum als bekannte Katheter zur herkömmlichen Durchführung der Aortenklappenablation besitzen kann und es ermöglicht, eine größere Anzahl oder andere und größere Instrumente durch den Hohlraum in den Arbeitsbereich einzubringen.

## Patentansprüche

1. Ballonkatheter (1) zum Abdichten von Blutgefäßen und Herzkammern mit wenigstens einer mit einer ersten Zuleitung (10) verbundenen aufblasbaren Kammer (20),
**gekennzeichnet durch**
eine an die wenigstens eine aufblasbare Kammer (20) angrenzende Einheit (40), die
- mit der Kammer (20) einen Hohlraum, in den eine Unterdruckleitung (30) mündet, bildet, wobei zwischen den den Hohlraum bildenden Wandungen Abstandselemente (60, 70) vorgesehen sind, und
- zum Ansaugen des Ballonkatheters (1) an das Blutgefäß oder die Herzkammer auf ihrer Außenseite wenigstens in einem begrenzten Bereich (50) gasdurchlässig ist.

2. Ballonkatheter (1) nach Anspruch 1, **dadurch gekennzeichnet, dass** die Einheit (40) die aufblasbare Kammer (20) vollständig umfasst.

3. Ballonkatheter (1) nach einem der vorhergehenden Ansprüche, **dadurch gekennzeichnet, dass** die Unterdruckleitung (30) die erste Zuleitung (10) umfasst.

4. Ballonkatheter (1) nach einem der vorhergehenden Ansprüche, **dadurch gekennzeichnet, dass** die Abstandselemente (60, 70) von der Unterdruckleitung (30) zu dem wenigstens einen begrenzten, gasdurchlässig ausgebildeten Bereich (50) führende Leitungen bilden.

5. Ballonkatheter (1) nach einem der vorhergehenden Ansprüche, **dadurch gekennzeichnet, dass** der wenigstens eine begrenzte, gasdurchlässig ausgebildete Bereich (50) wenigstens eine Pore besitzt.

6. Katheter mit wenigstens einem Hohlkanal (110) und wenigstens zwei, in Katheterlängserstreckung am distalen Katheterbereich voneinander beabstandeten Dilatationseinheiten (120a, 1),
**dadurch gekennzeichnet, dass**
wenigstens eine der Dilatationseinheiten (120a, 1) ein Ballonkatheter nach einem der Ansprüche 1 bis 5 ist.

## Claims

1. Balloon catheter (1) for sealing blood vessels and ventricles having at least one inflatable chamber (20) connected to a first feed line (10), **characterized by** a unit (40) adjacent to the at least one inflatable chamber (20) and which together with said chamber (20) forms a cavity into which issues a vacuum line (30), spacing elements (60, 70) being provided between the walls forming the cavity and for the suction of the balloon catheter (1) onto the blood vessel or ventricle at least a limited area (50) of the outside thereof is permeable to gas.

2. Balloon catheter (1) according to claim 1, **characterized in that** the unit (40) completely comprises the inflatable chamber (20).

3. Balloon catheter (1) according to one of the preceding claims, **characterized in that** the vacuum line (30) comprises the first feed line (10).

4. Balloon catheter (1) according to one of the preceding claims, **characterized in that** the spacing elements (60, 70) form lines leading from the vacuum line (30) to the at least one restricted, gas-permeable area (50).

5. Balloon catheter (1) according to one of the preceding claims, **characterized in that** the at least one restricted, gas-permeable area (50) has at least one pore.

6. Catheter having at least one hollow channel (110) and at least two dilation units (120a, 1) spaced from one another in catheter longitudinal extension at the distal catheter area, **characterized in that** at least one of the dilation units (120a, 1) is a balloon catheter according to one of the claims 1 to 5.

## Revendications

1. Cathéter à ballonnet (1) destiné à étancher des vaisseaux sanguins et des ventricules, comportant au moins une chambre gonflable (20) reliée à une première conduite d'alimentation (10),
**caractérisé par**
une unité (40) adjacente à ladite au moins une chambre gonflable (20), qui
- forme avec la chambre (20) une cavité dans laquelle débouche une conduite à dépression (30), des éléments d'écartement (60, 70) étant prévus entre les parois formant la cavité, et
- est perméable aux gaz sur son côté extérieur, au moins dans une zone limitée (50), pour attirer par succion le cathéter à ballonnet (1) contre le vaisseau sanguin ou le ventricule.

2. Cathéter à ballonnet (1) selon la revendication 1, **caractérisé en ce que** l'unité (40) entoure complètement la chambre gonflable (20).

3. Cathéter à ballonnet (1) selon l'une des revendications précédentes, **caractérisé en ce que** la conduite à dépression (30) entoure la première conduite d'alimentation (10).

4. Cathéter à ballonnet (1) selon l'une des revendications précédentes, **caractérisé en ce que** les éléments d'écartement (60, 70) forment des conduites menant de la conduite à dépression (30) à ladite au moins une zone (50) limitée, perméable aux gaz.

5. Cathéter à ballonnet (1) selon l'une des revendications précédentes, **caractérisé en ce que** ladite au moins une zone (50) limitée, perméable aux gaz possède au moins un pore.

6. Cathéter avec au moins un canal creux (110) et au moins deux unités de dilatation (120a, 1) distantes l'une de l'autre en extension longitudinale dans la zone distale du cathéter,
**caractérisé en ce**
**qu'**au moins une des unités de dilatation (120a, 1) est un cathéter à ballonnet selon l'une des revendications 1 à 5.
